# EUROPEAN PATENT SPECIFICATION

(11) **EP 0 370 083 B1**
(45) Date of publication and mention of the grant of the patent: **01.06.1994**
(21) Application number: 89904467.1
(22) Date of filing: 22.03.1989
(51) Int. Cl.: F16J 15/40

(54) **SEAL FOR ROTATABLE SHAFT**
DICHTUNG FÜR EINE ROTIERENDE ACHSE
JOINT POUR UN ARBRE ROTATIF

(30) Priority: 25.03.1988 US 150222
(43) Date of publication of application: 30.05.1990
(73) Proprietor: AMOCO CORPORATION, Chicago, IL 60680-0703 (US)
(72) Inventor: RICHARDS, Hiram, Bryant, Williford, AR 72482 (US)
(74) Representative: Laredo, Jack Joseph
(86) International application number: US8901222
(87) International publication number: WO8909357

(56) References cited:
- EP-A- 0 222 500
- DE-A- 3 420 523
- GB-A- 705 444
- GB-A- 2 032 920
- US-A- 3 871 666
- US-A- 3 979 128
- US-A- 4 114 902
- US-A- 4 371 176
- US-A- 4 501 431

## Description

### Technical Field

This invention relates to seal devices, and, in particular, to a dry seal about a rotatable shaft.

### Background of the Invention

In rotary pumps, fans, compressors, agitators, etc., a shaft projects through a casing in a region known as the "stuffing box" or "packing box". These terms are derived from the fact that in order to separate two environments, namely, that within the pump, fan, compressor, agitator, etc., and the atmosphere, and to prevent leakage of one environment into the other, material had to be stuffed or packed around the shaft where it passed through its casing. Soft packing materials have been employed for this purpose.

Presently, mechanical seals are commonly used for sealing against liquids when using a rotating shaft. These seals consist of radial planar surfaces which are normal to the shaft axis. The surfaces cooperate to function as a bearing.

A labyrinth seal is a type of mechanical seal. This seal is not fluid-tight but limits leakage by means of a tortuous path and induced turbulence. The seal consists of a ring or series of rings about the shaft which provide successive expansion of the fluid. As the differential pressure across any individual restriction is small, total leakage is minimized. These rings have a shaft clearance which is constantly maintained. Illustrative of labyrinth seals is British Patent No. 705,444 to Allen et al.

When no external leakage is permissible, purging with an inert gas is usually provided. Illustrative of such seals are those shown in U.S. Patent No. 3,071,384 to Friberg. Such seals utilize a constant flow of inert gas in combination with a vacuum to prevent leakage along a shaft (Column 5, lines 30-74). This requires a clearance between the spaced sections of the seal and the rotor and stator.

Alternatively, a liquid can be used as the purge medium to prevent leakage past the shaft and to provide lubricity. These seals are expensive, however. Furthermoe, seals are time-consuming to install, thus excessive downtime of the equipment is required when such a seal is to be replaced. They are also undesirable for a number of other reasons. Often, the liquid purge medium either leaks into the environment or the pump, turbine or the like, consequently the environment, the medium being pumped, or the equipment itself becomes contaminated. The liquid purge medium also can, and does, leak into the atmosphere, and may present undesirable safety conditions including potential fire hazards.

In addition, many of the prior art seals require the device operated upon by the shaft be disassembled to replace the seal. This is undesirable because of the additional man-hours and downtime required to disassemble and reassemble the device.

Accordingly, it is desirable to provide a seal which is dry, prevents leakage of a fluid along the shaft, and is easy to install. The present invention satisfies these desires.

### Summary of the Invention

According to the present invention, there is provided a labyrinth type seal (10) for a rotatable shaft (36) comprising scaling elements which consist of radial planar surfaces normally to the shaft axis and which cooperate to provide for successive expansion of fluid within the seal, said seal being characterised by:
at least one axially monolithic, elastomeric sealing element (11) having a shaft side (12) and a casing side (14), and provided on the shaft side thereof with a plurality of radially inwardly extending circumferential finds (20) each tapering toward an apex;
each of said fins (20) having an asymmetric cross-section in a plane passing through the axis of said shaft (36);
two of said fins (20) being spaced from one another and defining therebetween a circumferential purge chamber (32);
the apex of each of said fins (20) extending toward said shaft (36) when the seal (10) is positioned about the shaft (36);
a plurality of adjacent fins (20) provided on each side of said purge chamber (32) defining therebetween a circumferential sealing chamber (24); said adjacent fins (20) having apices (22) pointing in the same axial direction away from said purge chamber (32) and said sealing chamber (24) having an asymmetric cross-section (21) in a plane passing through the axis of said shaft (36).

The present invention contemplates a seal, for a rotatable shaft, that can be replaced relatively inexpensively and easily. This seal is monolithic along the shaft and utilizes a plurality of circumferential fins having an asymmetric cross-section to define, together with the shaft, plural sealing chambers and prevent or minimize undesirable leakage.

The seal of the present invention is a dry seal which includes a single, elongated axially monolithic elastomeric sealing element, or a plurality of such sealing elements, each having a shaft side and a casing side. The shaft side is provided with a plurality of radially inwardly extending circumferential fins, each fin tapering towards an apex. The cross-section of each fin in a plane that includes the axis of the shaft is asymmetric. Two spaced fins define therebetween a circumferential purge chamber having at least one inlet which extends through the elastomeric sealing element to the purge chamber. The apices of the fins on each side of the purge chamber point in an axial direction away from the purge chamber. Each fin apex extends to the shaft, with minimal or substantially no clearance, when the seal is positioned about the shaft. Optionally, a plurality of adjacent fins having apices pointing in the same axial direction away from the purge chamber are provided on each side of the purge chamber to define therebetween a sealing chamber or chambers.

In use, the seal is first positioned about the shaft. Inert gas is then introduced under pressure through the purge chamber inlet into the purge chamber. Inert gas thus flows into and purges any adjacent sealing chambers defined between the fins. In use, the principal flow of the purge gas is toward the interior of the vessel from which the shaft extends.

The seal of the present invention is easily installed with a minimum of downtime. Furthermore, inasmuch as the seal is a "dry" seal, the undesirable safety conditions of the prior art liquid purge seals are overcome without the loss of lubricity. In addition, the selection of a seal material such as a fluoroelastomer further assists in minimizing friction between the seal and the shaft.

Numerous other advantages and features of the present invention will become readily apparent from the following detailed description of the invention, the accompanying examples, the drawings, and the appended claims.

### Brief Description of the Drawings

In the drawings:
FIGURE 1 is a perspective view in partial section of a seal embodying the present invention;
FIGURE 2 is a longitudinal sectional view of a seal embodying the present invention secured to a seal casing and positioned about a rotatable shaft; and
FIGURE 3 is an expanded perspective view showing unassembled elements of a split seal embodying the present invention positioned about a rotatable shaft, the shaft being shown partially in phantom.

### Description of Preferred Embodiments

As used in the ensuing specification and claims, the term "axially monolithic" means that in the axial direction along the shaft there is a single elongated sealing element. However, about the periphery of the shaft the seal may be constituted by one or more of such axially monolithic sealing elements.

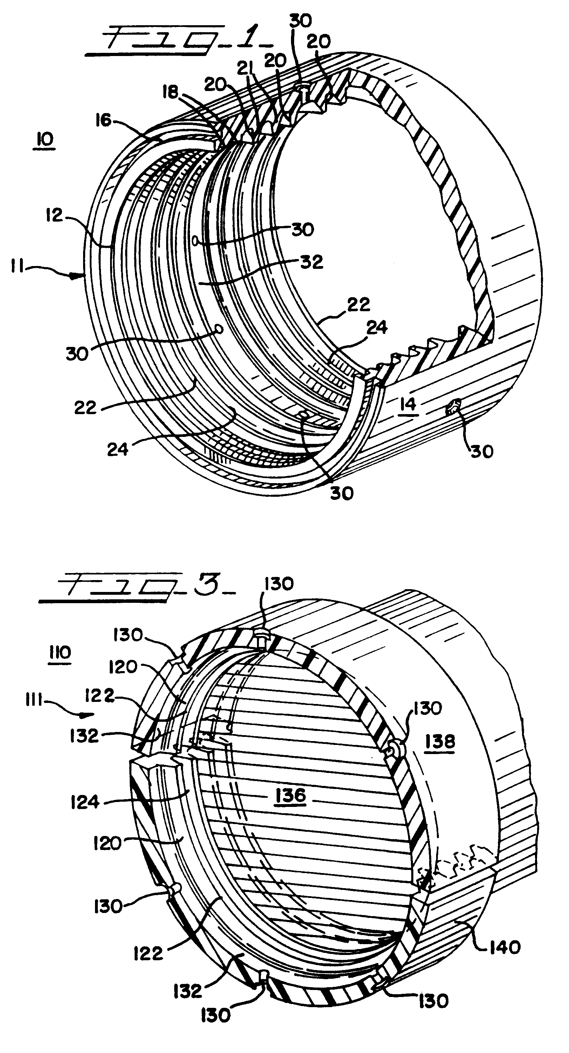

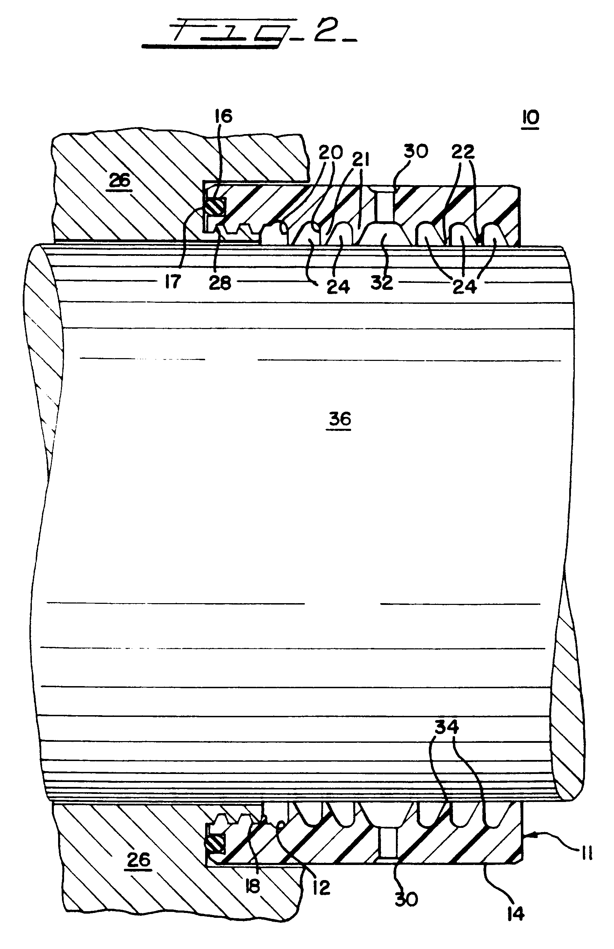

An embodiment of the present invention, a seal 10 for a rotatable shaft 36, is shown in FIGURES 1 and 2. The seal 10 includes an elastomeric sealing element 11 which is axially as well as circumferentially monolithic, and has a shaft side 12 and a casing side 14. The shaft side 12 is provided with a plurality of radially inwardly extending circumferential fins 20 that define, together with the shaft 36, a circumferential purge chamber 32 and one or more circumferential sealing chambers 24 about the shaft 36 on each side of purge chamber 32.

Each fin 20 tapers towards an apex 22. The fins 20 have an asymmetric cross-section 21 in a plane that includes the axis of the shaft 36. The angle at which the apices 22 of the fins 20 point in an axial direction away from the purge chamber 32 usually is about 25° to about 40° and preferably is about 30°. The depth of the fins 20 usually is about 7 mm (0.275 inch) to about 9.5 mm (0.375 inch), preferably about 7.6 mm (0.3 inch). The spacing between adjacent fins 20 usually is about 2.54 mm (0.1 inch) to about 19 mm (0.75 inch), preferably about 10 mm (0.4 inch).

Two spaced fins with apices pointing away from one another define therebetween a circumferential purge chamber 32 which is in communication with an inert gas source (not shown) via at least one purge chamber inlet 30. Preferably, there are provided six purge chamber inlets 30 spaced substantially equally about the purge chamber 32 in a plane perpendicular to the axis of the shaft 36.

A plurality of fins is provided on each side of the purge chamber 32 so that two adjacent fins 20 having apices 22 pointing in the same axial direction away from the purge chamber 32 together define a circumferential sealing chamber 24. At least one sealing chamber 24 is adjacent to the purge chamber 32 on each side thereof, and more preferably there are at least three such sealing chambers. The sealing chamber 24 thus defined has an asymmetric cross-section in a plane passing through the axis of the shaft 36 as shown in FIGURE 2. The volume ratio of sealing chamber-to-purge chamber usually is in the range of about 0.25:1 to about 2:1. Preferably, the volume of each sealing chamber is about one-half of the volume of the purge chamber; however, the volume of each individual sealing chamber of the seal need not be the same.

Apex 22 of each fin 20 extends toward rotatable shaft 36. While the apices 22 need not contact the shaft 36, the clearance between apices 22 and shaft 36 is minimal, i.e., 1,3 mm (0.050 inch), or less. This provides improved overall sealing characteristics. Since at most only a relatively small surface area of the seal 10 actually contacts the shaft 36 at a band contact area 34, the total contact surface between the seal and the shaft is relatively small in any event, thus minimizing friction. Friction can be further reduced by manufacturing the elastomeric sealing element from a filled fluoropolymer having a relatively low coefficient of friction vis-a-vis the shaft. Preferred filled fluoropolymers are carbon-filled fluoropolymers of the type disclosed in U.S. Patent No. 2,891,921 to Kumnick et al. These fluoropolymers are commercially available.

An O-ring receiving groove 16 is defined by the elastomeric sealing element 11 in one end face thereof. The O-ring 17 received within groove 16 prevents leakage between the seal 10 and the seal casing 26.

The elastomeric sealing element 11 is also internally threaded at one end to receive threads 28 of the seal casing 26 for securing the seal 10 to the seal casing 26.

FIGURE 3 illustrates an alternative embodiment of the seal 110. In this alternative embodiment, the elastomeric sealing element 111 is generally of the same external and internal configuration as sealing element 11 but is comprised of two complementary, axially monolithic portions 138 and 140 positioned about the shaft 136. Thus, while the sealing element 111 is axially monolithic along the shaft 136, it is not circumferentially monolithic about the shaft 136. The axially monolithic portions 138 and 140 complement one another and completely surround the shaft when installed. In this view, the seal 110 is shown unassembled. Therefore, the apex 122 of fin 120 is not shown in contact with the shaft 136 although in an assembled seal apex 122 may bear against the shaft 136.

Also shown in FIGURE 3 are the purge chamber 132 and the six purge chamber inlets 130 spaced substantially equally about the periphery of purge chamber 132 in a plane perpendicular to the axis of the shaft 136. This arrangement of the purge chamber inlets 130 is the same as the respective arrangement in the embodiment of FIGURES 1 and 2.

The use of a preferred embodiment of the present invention will be discussed in connection with FIGURES 1 and 2. The alternative embodiment of FIGURE 3 is used in a like manner but provides additional time savings during installation by virtue of its two complementary portions.

The seal 10 is first positioned about shaft 36 and then the threads 28 of the seal casing 26 are matingly engaged with internal threads 18 of seal 10. The O-ring 17 in the O-ring receiving groove 16 is thereby urged into contact with the seal casing 26 to prevent leakage between the seal 10 and the seal casing 26.

Inert gas from a source (not shown) is introduced into the circumferential purge chamber 32 through the purge chamber inlet 30. Preferably, six purge chamber inlets 30 are utilized to provide equal distribution of the inert gas. After all sealing chambers 24 are purged by the introduction of inert gas, the inert gas flow is maintained in a direction along the shaft and toward the interior of the vessel from which the shaft extends.

The fins 20 in cooperation with the purge chamber 32 and the sealing chambers 24, and coacting with the shaft 36, prevent undesirable leakage of fluid along the shaft 36. Thus, contamination of the environment or equipment is prevented or at least minimized. Because of the shape and orientation of the fins 20, any external pressure exerted thereupon in an axial direction is resisted.

The present invention can be molded or machined from a material of construction which is compatible with the operating environment, as desired. Particularly preferred as materials of construction are the aforementioned filled fluoropolymers.

The seal of the present invention can be installed with minimal downtime as disassembly of the device operated upon by the shaft usually is not required.

The seals of this invention are suitable for use in the process for preparing aromatic carboxylic acids by the liquid-phase oxidation of alkyl substituted aromatic hydrocarbons. In particular, the seals of this invention are useful in the preparation of terephthalic acid by the liquid-phase oxidation of para-xylene. Terephthalic acid is an important commercial chemical used in the preparation of polyester materials as well as other polymeric materials.

In the process of producing terephthalic acid from para-xylene relatively pure para-xylene (p-xylene) is required. One particularly suitable method of preparing pure p-xylene comprises the low-temperature crystallization of p-xylene from a mixture of p-xylene, m-xylene and o-xylene followed by a separation of the crystallized p-xylene from the remaining mother liquor by a low-temperature separation process wherein centrifugation is a preferred method for separating the crystallized p-xylene from the liquid mother liquor.

The seals of this invention are particularly suitable for use in the centrifuges used for recovering the pure, crystallized p-xylene. In these centrifuges the liquid xylene mother liquor must not escape along the centrifuge shaft and beyond the centrifuge casing. Otherwise, the liquid xylene leakage would present a serious fire hazard in the manufacturing plant. Furthermore, it is essential to minimize the time the centrifuge is non-operational by providing parts for the centrifuge that do not malfunction and by providing parts that do not frequently wear out.

The seals of this invention meet all of the aforementioned criteria. Due to their labyrinth design, gas purge feature and purge chamber, they prevent the leakage of the flammable xylene mixture along the centrifuge shaft. The seals of this invention are more durable than the conventional mechanical seals thereby reducing the need for replacement due to malfunction and wear. Furthermore, due to their design, and particularly the split seal design, the seals of this invention are more easily and more rapidly replaced than the conventional prior art seals.

The next step in the preparation of terephthalic acid requires the liquid-phase oxidation of the purified p-xylene. In this step, purified p-xylene is oxidized using an oxygen containing gas, such as air, in a liquid medium comprising an aliphatic low molecular weight carboxylic acid solvent, in the presence of a catalyst comprising cobalt, manganese and bromine components, at a temperature of from 100°C to about 350°C, and at a pressure sufficient to maintain at least a portion of the solvent in the liquid phase, to form a reaction product mixture containing terephthalic acid. During this liquid-phase oxidation, the two methyl groups on the p-xylene aromatic nucleus are both oxidized to carboxylic acid groups. In this process acetic acid is a preferred reaction solvent and hydrogen bromide is a preferred source of the bromine catalyst component. The manganese and cobalt catalyst components may be obtained from a variety of sources, however, the acetate salts of these metals are preferred.

The liquid-phase oxidation to p-xylene to terephthalic acid is accomplished in a reactor vessel comprising an inlet or inlets for air or other oxygen containing gas, baffles along the inside walls of the vessel to promote agitation, and an agitator to vigorously agitate the oxidation reaction mixture during the course of the oxidation reaction. The agitator comprises a shaft extending through the reactor vessel wall with the end of the shaft located within the vessel having one or more impellers secured to the shaft to effect the agitation of the reaction mixture upon the rotation of the shaft. The other end of the shaft is connected to a means for rapidly rotating the agitator shaft. At the point where the shaft passes through the reactor vessel wall a seal is required to prevent leakage of the gaseous or liquid-phase components of the reaction mixture. The seals of this invention are particularly useful for providing an effective seal between the agitator shaft and the wall of the reactor vessel. The seals of this invention are easily replaced, particularly the split seal of this invention, and they have an exceedingly long useful life. Due to their design, they are not susceptible to frequent malfunction. In addition, the elastomeric seal materials are inert to the corrosive oxidation reaction mixture which comprises acetic acid and other acidic components such as hydrogen bromide. The seals are, therefore, not prone to wear or malfunction due to corrosion.

Subsequent to the liquid-phase oxidation reaction, the oxidation reaction mixture is cooled, or treated in some other manner, to effect the crystallization of terephthalic acid from the oxidation reaction mixture thereby producing a slurry of terephthalic acid in the oxidation reaction mother liquor. The crude terephthalic acid is subsequently physically separated from the mother liquor and centrifugation is particularly suitable means for separating the crude terephthalic acid from the oxidation reaction mother liquor.

The seals of this invention are particularly suitable for use in the centrifuges used to recover the crude terephthalic acid from the oxidation reaction mother liquor. The seals prevent the leakage of the mother liquor along the seal shaft extending out from the centrifuge casing. The oxidation reaction mother liquor comprises acetic acid and other acidic components, and in order to eliminate a potential hazard leakage from the centrifuge must be prevented. In addition, the elastomeric materials used for the construction of the seals of this invention are not susceptible to corrosion. The oxidation reaction mother liquor, particularly if held at elevated temperatures during the centrifugation process, is corrosive due to its acidic components. The elastomeric materials used for the seals of this invention are superior to the mechanical seals of the prior art in withstanding the exposure to the corrosive oxidation reaction mother liquor.

The seals of this invention when placed on centrifuges used for separating terephthalic acid from oxidation reaction mother liquor are also more durable and more easily replaced than the conventional prior art seals thereby reducing the time the centrifuges are non-operational. This results in a significant cost savings to the manufacturing process. The split seals of this invention are particularly preferred since they are most easily replaced.

An additional process step in the manufacture of terephthalic acid is a purification step wherein crude terephthalic acid is converted to purified terephthalic acid by exposing an aqueous solution of the crude terephthalic acid to hydrogen over a catalyst metal selected from a Group VIII metal of the Periodic Table of Elements. Preferably, the Group VIII is supported on a support material. The hydrogen treatment is carried out at a temperature of about 100°C to about 350°C and at a pressure sufficient to maintain the solution of crude terephthalic acid substantially in the liquid phase. Upon cooling the solution treated in this manner, substantially purified terephthalic acid crystallizes. The purified terephthalic acid is separated from the water solvent and centrifugation is a particularly suitable means for effecting this separation. The seals of this invention are advantageously employed in the centrifuge apparatus for this process due to the long service life of the seals and also because they are easily replaced. The split seals are particularly advantageous since they are most easily and most rapidly replaced.

While the oxidation processes described above are for the oxidation of p-xylene to terephthalic acid, similar processes are applicable for the liquid-phase oxidation of all alkyl substituted aromatic hydrocarbons as, for example, the oxidation of meta-xylene to isophthalic acid, the oxidation of o-xylene to phthalic acid or anhydride, the oxidation of pseudocumene to trimellitic acid or anhydride, the oxidation of durene to pyromellitic acid or anhydride, of mesitylene to trimesic acid, of 2,6-dimethylnaphthalene to 2,6-naphthalenedicarboxylic acid, and of other alkyl substituted aromatic compounds wherein the alkyl groups are oxidizable to carboxylic acid groups using liquid-phase oxidation conditions. The seals of this invention are useful in the equipment used in all of these processes.

Additionally, the seals of this invention can be used in other machinery or equipment within the plants that manufacture aromatic carboxylic acids wherein the equipment or machinery contains a rotating shaft and where it is beneficial to provide for a durable, leak-preventing, easily replaceable seal between the rotating shaft and the housing or casing of the equipment or machinery, such as, for example, dryer circulation fans, oxidation process air compressors, rotary pumps and oxidation catalyst recovery centrifuges.

## Claims

1. A labyrinth type seal (10) for a rotatable shaft (36) comprising sealing elements which consist of radial planar surfaces normal to the shaft axis and which cooperate to function as a bearing and which provide for successive expansion of fluid within the seal, said seal being characterised by:
at least one axially monolithic, elastomeric sealing element (11) having a shaft side (12) and a casing side (14), and provided on the shaft side thereof with a plurality of radially inwardly extending circumferential fins (20) each tapering toward an apex;
each of said fins (20) having an asymmetric cross-section in a plane passing through the axis of said shaft (36);
two of said fins (20) being spaced from one another and defining therebetween a circumferential purge chamber (32);
the apex of each of said fins (20) extending toward said shaft (36) when the seal (10) is positioned about the shaft (36);
a plurality of adjacent fins (20) provided on each side of said purge chamber (32) so that two adjacent fins (20) together define therebetween a circumferential sealing chamber (24); said adjacent fins (20) having apices (22) pointing in the same axial direction away from said purge chamber (32) and said sealing chamber (24) having an asymmetric cross-section (21) in a plane passing through the axis of said shaft (36).

2. The seal of Claim 1 further provided with at least one purge chamber inlet (30) defined by and extending through said elastomeric sealing element (11) to said purge chamber (32).

3. The seal of Claim 2 wherein six purge chamber inlets (30) are spaced substantially equally about the periphery of said purge chamber (32).

4. The seal of Claim 1 wherein said elastomeric sealing element (111) comprises a plurality of complementary portions (138, 140).

5. The seal of Claim 1 wherein said elastomeric sealing element (11) is composed of a filled fluoropolymer.

6. The seal of Claim 5, wherein said filled fluoropolymer is a carbon-filled fluoropolymer.

7. The seal of Claim 1 wherein said elastomeric sealing element (11) is provided with an O-ring receiving groove (16) at one end face of the sealing element (11).

8. The seal of Claim 1 wherein said elastomeric sealing element (11) is provided with a plurality of circumferential internal threads at one end of said elastomeric sealing element (11), said internal threads being radially inwardly extended on the shaft side of said elastomeric sealing element (11).

9. A seal according to Claim 1, wherein said elastomeric sealing element (11) is provided with an O-ring receiving groove (16) at one end face of said elastomeric sealing element (11); and
said elastomeric sealing element (11) is provided with a plurality of circumferential internal threads at that end of said elastomeric sealing element (11) which has said O-ring receiving groove (11), said internal threads being radially inwardly extended on the shaft side (12) of said elastomeric sealing element (11).

10. The seal of any of Claims 1 to 9, wherein a centrifuge equipped with such seals (10) is used in a process for separating a crystallized terephthalic acid from the remaining reaction product mixture.

11. The seal of any of Claims 1 to 9, wherein a centrifuge equipped with such seals (10) is used in a process for separating a purified terephthalic acid from the remaining solution.

12. The seal of any of Claims 1 to 9, wherein said seal (10) is used for sealing the shaft of an agitator to a reactor vessel, the liquid-phase oxidation of a process for preparing terephthalic acid being carried out in said reactor vessel.

## Patentansprüche

1. Eine Dichtung des Labyrinthtyps (10) für eine rotierende Welle (36), die Dichtungselemente umfaßt, welche aus radialen, planaren Oberflächen senkrecht zu der Wellenachse bestehen, welche zusammen als ein Lager funktionieren und welche für eine schrittweise Expansion des Fluids in der Dichtung sorgen, wobei die genannte Dichtung dadurch charakterisiert wird, daß:
wenigstens ein axiales, monolithisches, elastomeres Dichtungselement (11) eine Wellenseite (12) und eine Gehäuseseite (14) hat und auf seiner Wellenseite mit einer Vielzahl von radial nach innen sich ausdehnenden umfangsseitigen Graten (20) versehen ist, die sich jeweils zu einem Scheitelpunkt hin verjüngen;
jeder der genannten Grate (20) einen asymmetrischen Querschnitt in einer Ebene hat, die durch die Achse der genannten Welle (36) geht;
zwei der genannten Grate (20) mit Abstand zueinander angeordnet sind und dazwischen eine umfangsseitige Auslaßkammer (32) definieren;
der Scheitelpunkt von jedem der genannten Grate (20) sich in Richtung der genannten Welle (36) ausdehnt, wenn die Dichtung (10) um die Welle (36) herum angeordnet wird;
eine Vielzahl von benachbarten Graten (20) die auf jeder Seite der genannten Auslaßkammer (32) zur Verfügung gestellt werden, so daß die zwei benachbarten Grate (20) zusammen dazwischen eine umfangsseitige Dichtungskammer (24) definieren, wobei die genannten benachbarten Grate (20) Scheitelpunkte (22) haben, welche in die gleiche axiale Richtung weg von der genannten Auslaßkammer (32) zeigen und die genannte Dichtungskammer (24) einen asymmetrischen Querschnitt (21) hat, in einer Ebene, die durch die Achse der genannten Welle (36) geht.

2. Die Dichtung nach Anspruch 1, weiterhin ausgestattet mit wenigstens einem Auslaßkammereingang (30), definiert durch und sich ausdehnend über das genannte elastomere Dichtungselement (11) zu der genannten Auslaßkammer (32).

3. Die Dichtung nach Anspruch 2, wobei sechs Auslaßkammereingänge (30) im wesentlichen gleichmäßig um den Rand der genannten Auslaßkammer (32) herum angeordnet sind.

4. Die Dichtung nach Anspruch 1, wobei das genannte elastomere Dichtungselement (111) eine Vielzahl von komplementären Teilstücken (138, 140) umfaßt.

5. Die Dichtung nach Anspruch 1, wobei das genannte elastomere Dichtungselement (11) sich aus einem gefüllten Fluorpolymer zusammensetzt.

6. Die Dichtung nach Anspruch 5, wobei das genannte gefüllte Fluorpolymer ein mit Kohlenstoff gefülltes Fluorpolymer ist.

7. Die Dichtung nach Anspruch 1, wobei das genannte elastomere Dichtungselement (11) an einer Endfläche des Dichtungselements (11) mit einer einen O-Ring aufnehmenden Rille (16) versehen ist.

8. Die Dichtung nach Anspruch 1, wobei das genannte elastomere Dichtungselement (11) an einem Ende des genannten elastomeren Dichtungselements (11) mit einer Vielzahl von umfangsseitigen inneren Gewindegängen versehen ist, wobei die inneren Gewindegänge sich auf der Wellenseite des genannten elastomeren Dichtungselements (11) radial nach innen ausdehnen.

9. Eine Dichtung gemäß Anspruch 1, wobei das genannte elastomere Dichtungselement (11) an einer Endfläche des genannten elastomeren Dichtungselements (11) mit einer einen O-Ring aufnehmenden Rille (16) versehen ist; und
das genannte elastomere Dichtungselement (11) an demjenigen Ende des genannten elastomeren Dichtungselements (11) mit einer Vielzahl von umfangsseitigen inneren Gewindegängen versehen ist, welches die den O-Ring aufnehmende Rille (16) hat, wobei sich die inneren Gewindegänge auf der Wellenseite (12) des genannten elastomeren Dichtungselements (11) radial nach innen ausdehnen.

10. Die Dichtung nach einem der Ansprüche 1 bis 9, wobei eine Zentrifuge, welche mit solchen Dichtungen (10) ausgerüstet ist, in einem Verfahren zur Trennung einer kristallisierten Terephthalsäure von der verbleibenden Reaktionsproduktmischung benutzt wird.

11. Die Dichtung nach einem der Ansprüche 1 bis 9, wobei eine Zentrifuge, welche mit solchen Dichtungen (10) ausgerüstet ist, in einem Verfahren zur Trennung einer gereinigten Terephthalsäure von der verbleibenden Lösung benutzt wird.

12. Die Dichtung nach einem der Ansprüche 1 bis 9, wobei die genannte Dichtung (10) benutzt wird, um die Welle eines Rührwerks zu einem Reaktorgefäß abzudichten, wobei die Flüssigphasenoxidation eines Prozesses zur Herstellung von Terephthalsäure in dem genannten Reaktorgefäß ausgeführt wird.

## Revendications

1. Joint d'étanchéité du type à labyrinthe (10) pour un arbre rotatif (36), comportant des éléments d'étanchéité qui comprennent des sur faces radiales planaires perpendiculaires à l'axe de l'arbre et coopèrent de manière à fonctionner en tant que palier et permettent une dilatation successive du fluide à l'intérieur du joint d'étanchéité, ledit joint d'étanchéité étant caractérisé par
au moins un élément d'étanchéité élastomère (11) monolithique axialement et possédant un côté (12) tourné vers l'arbre et un côté (14) situé côté boîtier, et qui comporte, sur son côté tourné vers l'arbre, une pluralité de nervures circonférentielles (20) dirigées radialement vers l'intérieur et qui se rétrécissent chacune en direction d'un sommet;
chacune desdites nervures (20) possède une section transversale asymétrique dans un plan passant par l'axe dudit arbre (36);
deux desdites nervures (20) étant espacées l'une de l'autre et définissant entre elles une chambre circonférentielle de purge (32);
le sommet de chacune desdites nervures (20) s'étendant en direction dudit arbre (36) lorsque le joint d'étanchéité (10) est positionné autour de l'arbre (36);
une pluralité de nervures adjacentes (20) prévues de chaque côté de ladite chambre de purge (32) de sorte que deux nervures adjacentes (20) définissent entre elles une chambre circonférentielle d'étanchéité (24); lesdites nervures adjacentes (20) comportant des sommets (22) dirigés dans la même direction axiale s'écartant de ladite chambre de purge (32), et ladite chambre d'étanchéité (24) possédant une section transversale asymétrique (21) dans un plan passant par l'arbre dudit arbre (36).

2. Joint d'étanchéité selon la revendication 1, comportant en outre au moins une entrée (30) pour la chambre de purge, définie par et s'étendant à travers ledit élément d'étanchéité élastomère (11) en direction de ladite chambre de purge (32).

3. Joint d'étanchéité selon la revendication 2, dans lequel six entrées (30) de la chambre de purge sont espacées d'une manière sensiblement égale sur la périphérie de ladite chambre de purge (32).

4. Joint d'étanchéité selon la revendication 1, dans lequel ledit élément d'étanchéité élastomère (111) comporte une pluralité de parties complémentaires (138,140).

5. Joint d'étanchéité selon la revendication 1, dans lequel ledit élément d'étanchéité élastomère (11) est constitué par un polymère fluoré chargé.

6. Joint d'étanchéité selon la revendication 5, dans lequel ledit polymère fluoré chargé est un polymère fluoré chargé de carbone.

7. Joint d'étanchéité selon la revendication 1, dans lequel ledit élément d'étanchéité élastomère (11) comporte une gorge (16) recevant un joint torique, dans une face d'extrémité de l'élément d' étanchéité (11).

8. Joint d'étanchéité selon la revendication 1, dans lequel ledit élément d'étanchéité (11) comporte, à l'une de ses extrémités, une pluralité de filetages internes circonférentiels, lesdits filetages internes s'étendant radialement vers l'intérieur du côté dudit élément d'étanchéité élastomère (11) tourné vers l'arbre.

9. Joint d'étanchéité selon la revendication 1, dans lequel ledit élément d'étanchéité élastomère (11) comporte dans une face d'extrémité, une gorge (16) recevant un joint torique; et
ledit élément d'étanchéité élastomère (11) comporte une pluralité de filetages intérieurs circonférentiels situés sur l'extrémité dudit élément d'étanchéité élastomère (11), qui comporte ladite gorge (11) recevant le joint torique, lesdits filetages intérieurs s'étendant radialement vers l'intérieur sur le côté (12), tourné vers l'arbre, dudit élément d'étanchéité élastomère (11).

10. Joint d'étanchéité selon l'une quelconque des revendications 1 à 9, dans lequel une centrifugeuse munie de tels joints d'étanchéité (10) est utilisée dans un procédé pour réaliser la séparation de l'acide téréphtalique cristallisé à partir du mélange restant du produit de réaction.

11. Joint d'étanchéité selon l'une quelconque des revendications 1 à 9, dans lequel une centrifugeuse munie de tels joints d'étanchéité (10) est utilisée dans un processus pour séparer un acide téréphtalique purifié par rapport au reste de la solution.

12. Joint d'étanchéité selon l'une quelconque des revendications 1 à 9, dans lequel ledit joint d'étanchéité (10) est utilisé pour étanchéifier l'arbre d'un agitateur vis-à-vis d'une enceinte de réacteur, l'oxydation de la phase liquide d'un processus servant à préparer de l'acide téréphtalique étant exécutée dans ladite enceinte de réacteur.
